# EUROPÄISCHE PATENTSCHRIFT

(11) **EP 0 541 049 B1**
(45) Veröffentlichungstag und Bekanntmachung des Hinweises auf die Patenterteilung: **28.01.1998**
(21) Anmeldenummer: 92118854.6
(22) Anmeldetag: 04.11.1992
(51) Int. Cl.: A61F 9/02

(54) **Einrichtung zum Schutz gegen überhöhte optische Leistungs- und Energiedichten**
Device for protection against excessive optical power or energy densities
Dispositif pour la protection contre des densités de puissance et des quantités d'énergie lumineuse excessives

(30) Priorität: 07.11.1991 DE 4136588
(43) Veröffentlichungstag der Anmeldung: 12.05.1993
(73) Patentinhaber: Daimler-Benz Aerospace Aktiengesellschaft, 81663 München (DE)
(72) Erfinder: Lill, Ernst, Dr., W-8000 München 90 (DE)

(56) Entgegenhaltungen:
- EP-A- 0 349 665
- GB-A- 1 605 192
- US-A- 3 507 552
- US-A- 3 547 545
- US-A- 4 462 661
- US-A- 4 986 639

## Beschreibung

Die Erfindung bezieht sich auf eine Vorrichtung zum Schutz von Augen, elektrooptischen Sensoren und empfindlichen Materialien gegen überhöhte optische Leistungs- und Energiedichten.

Aufgrund ihrer hohen inheränten Empfindlichkeit sind Augen und elektrooptische Sensoren durch überhöhte optische Leistungs- und Energiedichte stark gefährdet. Als Schutzmaßnahmen sieht der Stand der Technik diverse passive und aktive Maßnahmen vor, wie beispielsweise Interferenzfilter, holographische Filter, mechanische Verschlüsse und selbstaktive optische Schalter.

Aus der US-A-3 547 545 ist es bekannt "photochrome" Substanzen einem Filter oder transparenten Film beizugeben, und aus der EP-A- 0 349 665 ist eine elektrooptische Linsenanordnung für einen Schweißhelm bekannt, die ein Flüssigkristall-Filter enthält.

Aus der US-A-3 507 552 ist es ebenfalls bekannt, 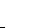 photochrome 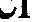 Substanzen einem Filter oder transparenten Element beizugeben die von einer warnsensorgesteuerten Lichtquelle angeregt wird, wobei es sich um Maßnahmen für den sichtbaren bzw. ultravioletten Spektralbereich handelt, also um einen sogenannten 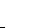 Farbfilter 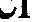 . Eine Abdeckung der für militärische Sensoren erforderlichen spektralen Schutzbereiche einschließlich des Infrarotbereiches ist mit derartigen Elementen nicht realisierbar.

Alle diese Ausführungsformen erbringen häufig nur einen unzureichenden Schutz, sei es, daß sie nur in einem eingeschränkten Spektralbereich wirksam sind oder die Sensorempfindlichkeit übermäßig reduziert wird. In einigen Fällen wird nach Abklingen der Bedrohung nicht angemessen auf Normalbetrieb geschaltet oder - insbesondere bei Laserpulsen - wird zu langsam reagiert. Ein weiteres Problem des Standes der Technik ist der hohe technische Aufwand - vornehmlich großes Gewicht und Volumen - bei vielen gängigen Schutzmaßnahmen.

Der Erfindung liegt die Aufgabe zugrunde, eine Vorrichtung der eingangs genannten Art zu schaffen, die einen zuverlässigen Schutz über einen großen Spektralbereich mit geringstem zeitlichen Verzug gewährleistet und sowohl zeitlich als auch bezüglich der Leistungs-/Energiedichte abgestimmt reagiert.

Diese Aufgabe wird die die im Anspruch 1 aufgezeigten Maßnahmen gelöst. In den Unteransprüchen sind Ausgestaltungen und Weiterbildungen angegeben und in der nachfolgenden Beschreibung sind Ausführungsbeispiele erläutert und in den Figuren der Zeichnung skizziert. Es zeigen:
- Fig. 1: ein Schemabild des Prinzips eines Ausführungsbeispieles der Schutzeinrichtung,
- Fig. 2: eine perspektivische Ansicht eines weiteren Ausführungsbeispiels der Schutzeinrichtung einer Spiegelkonfiguration bei longitudinaler Einkopplung der optischen Schaltenergie in schematischer Darstellung,
- Fig. 3: ein Schemabild einer Schutzeinrichtung mit Faserkoppler in schematischer Darstellung,
- Fig. 4: ein perspektivisches Schemabild eines Schutzsubstanzelementes für eine Schutzeinrichtung in Transmission bei transversaler Einkopplung.

Die Figuren zeigen nicht das zusätzliche, durch die Steuereinheit gesteuerte Schutzelement.

Der Grundgedanke der Erfindung ist darin zu sehen, daß auf der Erkenntnis basiert wird, wonach durch Licht, vorzugsweise Laserlicht, induzierte Veränderungen des optischen Transmissionsverhaltens unterschiedlicher Materialien hervorgerufen werden und daher der Übergang von maximaler Absorption, als auch der dazu inverse Effekt nutzbar sind. Zahlreiche Materialien weisen solche verwertbare Effekte auf. Als Beispiele seien aufgeführt:
Absorption von Farbstoffen,
das intensitätsabhängige Transmissionsverhalten von Halbleitersubstanzen,
die Plasmaerzeugung,
die Selbstdefokussierung,
die totale interne Reflexion,
die induzierte Streuung etc.

Der entscheidende Vorteil aller dieser Effekte ist in den kurzen Reaktionszeiten - die typisch bei < 1 ns liegen - zu sehen, also einer Eigenschaft, die insbesondere beim Schutz gegen Laserpulse geradezu unverzichtbar ist. Nun ist bekannt, daß das Auslösen dieser Effekte sehr hohe Leistungsdichten im Schutzmedium erfordert und zum Teil auch eine definierte Anregungswellenlänge.

Sollte die Störstrahlung den Schutz des betreffenden Sensors selbst auslösen, so mußte das Schutzmedium im Fokusbereich angeordnet werden. Dies erforderte wiederum eine zusätzliche Zwischenabbildung. Weiterhin ist anzuführen, daß selbst unter diesen Voraussetzungen der Schalt- und Abschwächungseffekt häufig erst bei Schwellwerten eintrat, die oberhalb der Gefährdungsgrenze des verwendeten Sensors lagen, also alle diese Schutzmaßnahmen des Standes der Technik als unbefriedigend bezeichnet werden müssen.

Eine Optimierung wird jedoch durch die vorgeschlagene Nutzung der vorstehend genannten Effekte dadurch erzielt, daß sie gezielt und bedarfsgerecht durch das Licht einer externen Strahlungsquelle, vorzugsweise einem Laser, ausgelöst werden. Leistungsdichte, Wellenlänge und Zeitverhalten der Strahlung werden somit durch die Wahl der Lichtquelle 12, deren Ansteuerung und der Einkopplung ihrer Strahlungsenergie optimal auf die Erfordernisse abgestimmt.

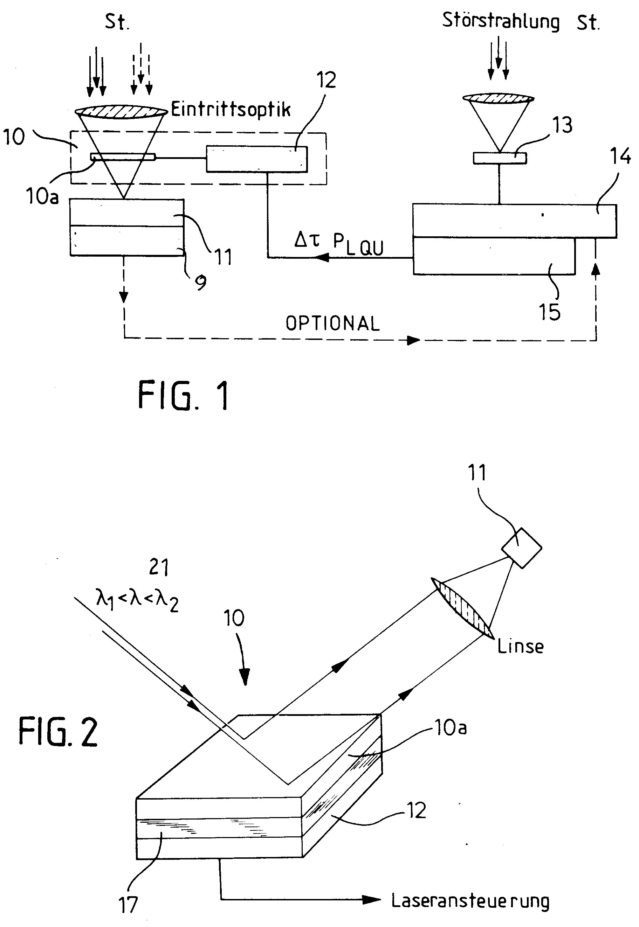

In der Fig. 1 ist eine Prinzipdarstellung eines Ausführungsbeispiels der vorgenannten Einrichtung gezeigt. Dieses Ausführungsbeispiel besteht aus dem zu schutzenden optischen Sensor 11 mit Detektor, in dessen Strahlengang das Schutzelement 10 angeordnet ist und einem Warnsensor 13, der mit einer Signalauswerteeinheit 14 und einer Steuereinheit 15 verbunden ist. Die Signalauswerteeinheit 14 führt eine sogenannte Bedrohungsanalyse aus, deren Meßergebnisse der Steuereinheit 15 übermittelt werden. Diese Steuereinheit 15 steuert und regelt die Lichtquelle 12 entsprechend der eingegangenen Bedrohungsdaten hinsichtlich Pulsdauer, Wiederholrate und Pulsleistung. Ein Mikroprozessor in der Steuereinheit 15 führt die Ablaufsteuerung der Sensorschutzeinrichtung systemgerecht durch.

Im ungefährdeten Betriebszustand ist die Lichtquelle 12 ausgeschaltet und somit die Transmission des Schutzelementes 10 maximal. Im Falle auftretender überhöhter, auf den Sensor 11 treffender, Lichtenergie- oder Leistungsdichte, induziert eine von einem Warnsensor 13 ausgelöste Lichtquelle 12 mit minimalstem Zeitverzug, im Schutzelement 10 eine der Störlichtintensität angemessene Abschwächung. Mit dem Abklingen der Gefährdung reduziert die Regelschaltung der Steuereinheit 15 die in das Schutzelement 10 eingekoppelte Lichtleistung, wodurch sich die Transmission wieder erhöht und das zu schützende Sensorsystem 11 praktisch verzugsfrei seine volle Empfindlichkeit wieder erreicht.

Im Vergleich zum zu schützenden Element - Sensor, Auge usw. - ist der Warnsensor 13 für eine erheblich höhere Stör- oder Zerstörschwelle ausgelegt. Der Warnsensor ist ein durch dieselbe Regelschleife gesteuertes Schutzelement zugeordnet, das ihn adaptiv schützt. Hierbei können zusätzlich die Signale des zu schützenden Sensors 11 für die Bedrohungsanalyse in der Signalauswerteeinheit 14 herangezogen werden.

Das Einkoppeln der Energie der Lichtquelle 12 - vorzugsweise ein Laser - kann sowohl transversal als auch longitudinal erfolgen, wobei die Lichtquelle 12 unmittelbar an dem Schutzsubstanzelement 10a angeordnet ist oder die Energie über einen Lichtleiter 16 oder einen optischen Strahlengang einspeisen kann.

In der Fig. 2 ist ein Ausführungsbeispiel für die longitudinale Einkopplung der optischen Schaltenergie skizziert. Bei diesem Ausführungsbeispiel wird das Schutzelement 10 in sogenannter Spiegelkonfiguration eingesetzt, die aus einem Schutzsubstanzelement 10a, einem vorzugsweise flächengleichen, darunter angeordneten, dichroitischen Spiegel 17 und einem damit verbundenen, zweidimensionalen Laserarray besteht.

Im ungestörten Zustand ist die Schutzsubstanz - beispielsweise Metalloxyd/Halbleiterverbindungen - für den Wellenlängenbereich des optischen Nutzsignals transparent. Das Nutzsignal wird somit am dichroitischen Spiegel 17 in Richtung zum Detektor 11 reflektiert. Für den Fall einer Sensorgefährdung wird die Lichtquelle 12 aktiviert, bei deren Wellenlänge λ_{L} der dichroitische Spiegel 17 durchlässig ist. Die optische Schaltenergie versetzt die Schutzsubstanz des Elementes 10a in einen stark absorbierenden Zustand, wodurch der Strahlengang zum Detektor 11 des Hauptsensors 9 unterbrochen wird und dieser somit vor Überlastung geschützt ist.

Falls die Lichtquelle vom Schutzsubstanzelement abgesetzt betrieben wird, kann die Einkopplung der Schaltenergie über Lichtleiter erfolgen.

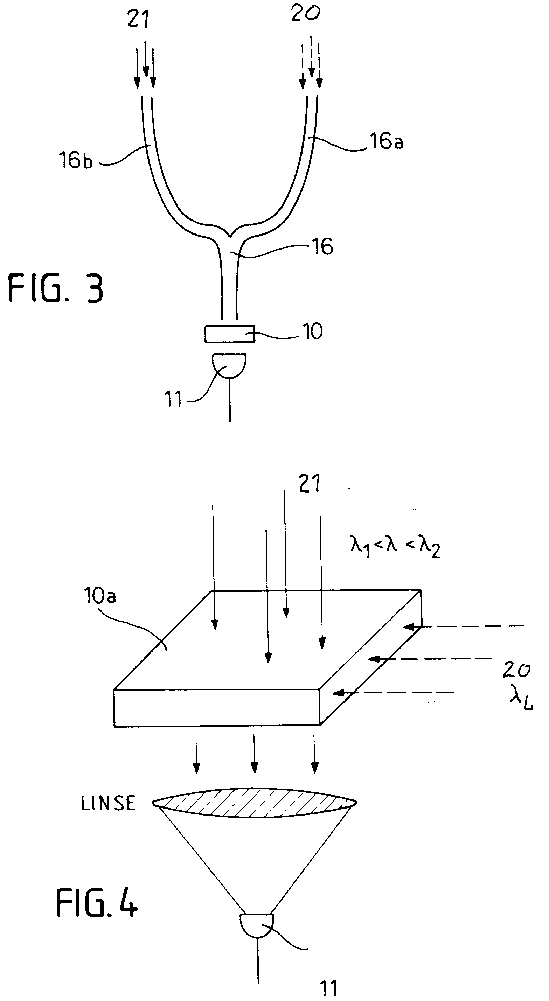

In Fig. 3 ist eine weitere Ausführungsvariante skizziert, bei der die optische Schaltenergie 20 und das optische Nutzsignal 21 mittels Faseroptik 16 überlagert und zusammen auf das Schutzelement 10 projiziert wird. Diese Ausführung hat den Vorteil, daß eine einfache Überlagerung der beiden Strahlanteile auf einem kleinen Bereich durchgeführt werden kann. Die Abmessungen des Schutzelementes können in diesem Fall äußerst kompakt und flächenmäßig minimiert ausgelegt werden.

In Fig. 4 wird der Fall einer transversalen Einkopplung der optischen Schaltenergie in das Schutzelement 10 dargestellt. In dieser Ausführungsform wird das Element 10a in Transmission betrieben.

Weitere Varianten der Schutzeinheit können linear oder als zweidimensionale Matrix aus Einzelelementen konzipiert sein, wobei jedes Element individuell geschaltet werden kann. Die longitudinale Einkopplung erfolgt hierbei über Glasfaserbündel oder Laserarray. Damit können partielle Überblendungen verhindert werden, falls der Detektor des Sensors 11 aus einer eindimensionalen oder zweidimensionalen Anordnung von Detektorelementen besteht.

Bei transversaler Einkopplung können die Laser an den Rändern des Schutzsubstanzelementes 10a angeordnet sein. Vorzugsweise wird die Wellenlänge des sogenannten Schaltlasers oder der Lichtquelle 12 so gewählt, daß die Funktion des Sensors 11 nicht beeinträchtigt wird.

## Patentansprüche

1. Vorrichtung zum Schutz von Augen, elektrooptischen Sensoren und empfindlichen Materialien gegen überhöhte optische Leistungs- und Energiedichten mittels eines in den Strahlengang des zu schützenden Objektes (11) - wie beispielsweise Auge, CCD-Kamera - angeordneten Schutzelementes (10), das aus einer im unbeleuchteten Zustand transparenten und im beleuchteten Zustand lichtabsorbierenden oder lichtstreuenden Schutzsubstanz (10a) besteht, wobei das Schutzelement (10) eine extern zum zu schützenden Objekt angeordnete Lichtquelle (12) für die Anregung der Schutzsubstanz (10a) umfaßt, wobei ein die einfallende Strahlung (St) detektierender Warnsensor (13) vorgesehen ist, der eine sehr hohe Stör- oder Zerstörschwelle aufweist und dessen Signale in eine Signalauswerteeinheit (14) für eine Bedrohungsanalyse eingehen, deren Ausgangssignale in eine Steuereinheit (15) zur Steuerung der Lichtquelle (12) hinsichtlich Pulsdauer, Wiederholfrequenz und Pulsleistung eingehen, und wobei dem Warnsensor (13) ihm zum adaptiven Schutz ein zusätzliches, durch die Steuereinheit (15) gesteuertes Schutzelement zugeordnet ist.

2. Vorrichtung nach Anspruch 1, **dadurch gekennzeichnet,** daß die Lichtquelle (12) ein Lasergerät ist, dessen Energie transversal oder longitudinal in die Schutzsubstanz (10a) eingekoppelt wird.

3. Vorrichtung nach Anspruch 1 oder 2, **dadurch gekennzeichnet,** daß die Lichtquelle (12) unmittelbar an der Schutzsubstanz (10a) angeordnet ist.

4. Vorrichtung nach einem der Ansprüche 1 bis 3, **dadurch gekennzeichnet,** daß der Lichtquelle (12) ein Lichtleiter (16) oder ein optischer Strahlengang zugeordnet ist.

5. Vorrichtung nach einem der Ansprüche 1 bis 4, **dadurch gekennzeichnet,** daß das Schutzelement (10) zur longitudinalen Lichteinkopplung in Sandwichbauweise aus der Schutzsubstanz (10a), einem darunterliegenden dichroitischen Spiegel (17) und einem dahinter anliegenden zweidimensionalen Laserarray als Lichtquelle (12) zusammengesetzt ist.

6. Vorrichtung nach einem der Ansprüche 1 bis 5, **dadurch gekennzeichnet,** daß den Lichtleitfasern (16a, 16b) ein Faserkoppler (16) zugeordnet ist, der die optische Schaltenergie der einen Faser mit dein optischen Nutzsignal der anderen Faser auf das Schutzelement (10) projiziert.

7. Vorrichtung nach einem der Ansprüche 1 bis 6, **dadurch gekennzeichnet,** daß bei transversaler oder longitudinaler Lichteinkopplung die Schutzsubstanz (10a) die optische Schaltenergie aufnimmt und das Schutzelement (10) in Transmission betrieben wird.

8. Vorrichtung nach einem der Ansprüche 1 bis 7, **dadurch gekennzeichnet,** daß sie aus Einzelelementen linear oder als zweidimensionale Matrix aufgebaut ist und diese Einzelelemente individuell schaltbar sind.

9. Vorrichtung nach einem der Ansprüche 1 bis 8, **dadurch gekennzeichnet,** daß die Lichtquelle (12) in ihrerWellenlänge, Leistung und Einschaltdauer an die störungsfreie Funktion des Hauptsensors anpassbar ist.

## Claims

1. Device for the protection of eyes, electro-optical sensors and sensors from excessive optical output and energy densities by means of a protective element (10) arranged in the beam path of the protected object (11), for example an eye or a CCD camera, composed of a protective substance (10a) which is transparent if not illuminated and light-absorbent or light-dispersing when illuminated, and for the purpose of activating the protective substance (10a) the protective element (10) includes a light source (12) arranged externally of the protected object, and a warning sensor (13) which detects incoming radiation (St) is provided, having a very high interference and destruction threshold and the signals of which are fed into a signal evaluation unit (14) for a threat analysis the output signals of which enter a control unit (15) to control the light source (12) relative to pulse duration, repeat frequency and pulse output, and the warning sensor (13) is, for the purpose of adaptive protection, associated with an additional protective element which is controlled by the control unit (15).

2. Device according to Claim 1, **characterised in that** the light source (12) is a laser unit the energy of which is transversally or longitudinally coupled into the protective substance (10a).

3. Device according to Claim 1 or 2, **characterised in that** the light source (12) is arranged directly on the protective substance (10a).

4. Device according to one of Claims 1 to 3, **characterised in that** the light source (12) is associated with an optical fibre (16) or an optical beam path.

5. Device according to one of Claims 1 to 4, **characterised in that** the protective element (10) is, for the purpose of longitudinal light introduction, assembled in sandwich fashion of the protective substance (10a), therebelow a dichroitic mirror (17) and therebelow a directly adjacent two-dimensional laser array as a light source (12).

6. Device according to one of Claims 1 to 5, **characterised in that** the optical fibres (16a, 16b) are associated with a fibre coupler (16) which projects the optical switch energy of the one fibre with the optical useful signal of the other fibre onto the protective element (10).

7. Device according to one of Claims 1 to 6, **characterised in that** during transversal or longitudinal light coupling the protective substance (10a) picks up the optical switch energy and the protective element (10) is operated in transmission.

8. Device according to one of Claims 1 to 7, **characterised in that** it is structured from individual elements linearly or as a two-dimensional matrix, and these individual elements can be switched individually.

9. Device according to one of Claims 1 to 8, **characterised in that** the light source (12) is in its wavelength, output and switch-on duration adaptable to interference-free function of the main sensor.

## Revendications

1. Dispositif pour la protection des yeux, de senseurs optoélectroniques et de matériaux sensibles contre des densités de puissance et d'énergie optique excessives à l'aide d'un élément de protection (10) placé sur le trajet des rayons de l'objct (11) à protéger - tel les yeux, une caméra CCD -, composé d'une substance de protection (10a) qui est transparente à l'état non éclairé et absorbe ou disperse la lumière à l'état éclairé, dispositif dans lequel l'élément de protection (10) comporte une source lumineuse (12) disposée à l'extérieur de l'objet à protéger, pour l'excitation de la substance de protection, dans lequel il est prévu un senseur d'alerte (13) qui détecte le rayon (St) incident, présente un seuil de perturbation ou de destruction très élevé et dont les signaux sont transmis à une unité (14) de traitement de signal pour une analyse de la menace, dont les signaux de sortie sont transmis à une unité de commande (15) pour la commande de la source lumineuse (12) du point de vue de la durée d'impulsion, de la fréquence de répétition et de la puissance d'impulsion, et dans lequel un élément de protection supplémentaire commandé par l'unité de commande (15) est associé au senseur d'alerte (13) à des fins de protection adaptative.

2. Dispositif selon la revendication 1, caractérisé par le fait que la source lumineuse (12) est un laser dont l'énergie est couplée transversalement ou longitudinalement dans la substance de protection (10a).

3. Dispositif selon la revendication 1 ou 2, caractérisé par le fait que la source lumineuse (12) est disposée directement contre la substance de protection (10a).

4. Dispositif selon l'une des revendications 1 à 3, caractérisé par le fait qu'un guide de lumière ou un trajet optique est associé à la source lumineuse (12).

5. Dispositif selon l'une des revendications 1 à 4, caractérisé par le fait que l'élément de protection (10) pour le couplage longitudinal de la lumière est formé d'une structure sandwich comprenant la substance de protection (10a), un miroir dichroïque (17) placé sous celle-ci, et d'un réseau laser bi-dimensionnel disposé sous l'ensemble en tant que source lumineuse (12).

6. Dispositif selon l'une des revendications 1 à 5, caractérisé par le fait qu'un coupleur (16) de fibre optique est associé aux fibres optiques (16a, 16b), lequel coupleur projette l'énergie optique de commande de l'une des fibres avec le signal utile de l'autre fibre sur l'élément de protection (10).

7. Dispositif selon l'une des revendications 1 à 6, caractérisé par le fait qu'en présence d'un couplage transversal ou longitudinal de la lumière, la substance de protection (10a) reçoit l'énergie optique de commande et que l'élément de protection (10) fonctionne en transmission.

8. Dispositif selon l'une des revendications 1 à 7, caractérisé par le fait qu'il est formé d'une matrice linéaire ou bi-dimensionnelle d'éléments individuels et que lesdits éléments individuels peuvent être commandés séparément.

9. Dispositif selon l'une des revendications 1 à 8, caractérisé par le fait que la source lumineuse (12) est adaptée du point de vue de sa longueur d'onde, de sa puissance et de sa durée de fonctionnement de manière à permettre un fonctionnement sans perturbation du senseur principal.
